# EUROPÄISCHE PATENTSCHRIFT

(11) **EP 0 381 942 B1**
(45) Veröffentlichungstag und Bekanntmachung des Hinweises auf die Patenterteilung: **25.05.1994**
(21) Anmeldenummer: 90100622.1
(22) Anmeldetag: 12.01.1990
(51) Int. Cl.: C07C 17/38, B01D 53/04

(54) **Verfahren und Vorrichtung zur Rückgewinnung von in einem Adsorber adsorbierten Perchloräthylen**
Process and device for the recovery of perchloroethylene absorbed by an adsorbent
Procédé et appareillage de récupération du perchloroéthylène adsorbé par un adsorbant

(30) Priorität: 07.02.1989 DE 3903522
(43) Veröffentlichungstag der Anmeldung: 16.08.1990
(73) Patentinhaber: PERO KG, 86343 Königsbrunn (DE)
(72) Erfinder: Erbel, Horst, D-8900 Augsburg 21 (DE)
(74) Vertreter: Charrier, Rolf, Dipl.-Ing.

(56) Entgegenhaltungen:
- DE-A- 3 048 649
- PATENT ABSTRACTS OF JAPAN, unexamined applications, Sektion C, Band 5, Nr. 35, 5. März 1981; THE PATENT OFFICE JAPANESE GOVERNMENT, Seite 66 C 46
- PATENT ABSTRACTS OF JAPAN, unexamined applications, Sektion C, Band 2, Nr. 59, 27. April 1978; THE PATENT OFFICE JAPANESE GOVERNMENT, Seite 458 C 78
- PATENT ABSTRACTS OF JAPAN, unexamined applications, Sektion C, Band 4, Nr. 26, 6. März 1980; THE PATENT OFFICE OF THE JAPANESE GOVERNMENT, Seite 106 C 1

## Beschreibung

Die Erfindung betrifft ein Verfahren zur Rückgewinnung von in einem Adsorber adsorbierten Perchloräthylen sowie eine Vorrichtung zur Durchführung des Verfahrens.

Nach dem DE-Patent 30 48 649 erfolgt die Rückgewinnung von an Aktivkohle adsorbierten chlorierten und/oder fluorierten Kohlenwasserstoffen dadurch, daß die Aktivkohle von Heißluft durchströmt wird, die eine Temperatur aufweist, die unter der Zersetzungstemperatur der Halogenwasserstoffe liegt. Die abströmende Luft wird sodann auf eine Temperatur abgekühlt, die unter dem Siedepunkt des Halogenkohlenwasserstoffs liegt, so daß eine Kondensation des Halogenkohlenwasserstoffs eintritt. Danach wird die Luft wieder erhitzt und von neuem durch die Aktivkohle geleitet. Am Ende dieses Kreislaufverfahrens weist jedoch die Luft eine Restmenge des Halogenkohlenwasserstoffs auf.

Nach einer Abänderung des Verfahrens wird die abströmende Luft weit unter den Siedepunkt des Halogenkohlenwasserstoffs abgekühlt. Durch die Kondensation des Kohlenwasserstoffs in der Abkühleinheit entsteht dort ein Unterdruck, der das Abziehen des Kohlenwasserstoffs begünstigt. Auch bei dieser Verfahrensvarianten weist die Luft eine Restmenge des Kohlenwasserstoffs auf.

Nach Ullmanns Enzyklopädie der Technischen Chemie, Band 2, 4. Auflage, Seiten 611, 612 ist es bekannt, einen Adsorber durch Drucksenkung zu regenerieren. Hierbei fällt bei sinkendem Druck das Desorbat als angereichertes Gemisch an. Bei niedrigem Druckniveau wird mit Spülgas gespült. Das

Spülgas z.B. Luft ist somit in starkem Maße mit dem desorbierten Stoff belastet.

Nach Patent Abstract of Japan, C, Vol.5, No.35, C46, Seite 66 (55-160730) wird zur Desorbierung von Methylbromid eine erste Aktivkohle, die eine hohe Adsorptionsfähigkeit bei geringen Methylbromidkonzentrationen aufweist, auf eine Temperatur von 50 bis 250°C erhitzt und an den Adsorberraum ein Druck von 26,7 bis 800 mbar angelegt. Das desorbierte Methylbromid wird abgekühlt und von einer zweiten Aktivkohle adsorbiert, die eine hohe Adsorptionsfähigkeit bei hohen Methylbromidkonzentrationen aufweist. Diese wird ebenfalls auf die vorgenannte Temperatur erhitzt und an den Adsorberraum für die zweite Aktivkohle der vorgenannte Unterdruck angelegt.

Nach Patent Abstract of Japan, C, Vol.2, No.59, C 78 Seite 458 (53-18504) wird zur Desorption von Halogenkohlenwasserstoffen Aktivkohle auf 50 bis 150°C erhitzt und an den Adsorberraum ein Unterdruck zwischen 6,66 und 666,6 mbar angelegt. Gleichzeitig wird Dampf, Luft oder ein Inertgas zugeführt. Das desorbierte Halogenkohlenwasserstoffgas wird durch Kühlen verflüssigt.

Es besteht die Aufgabe, das Verfahren so auszubilden, daß das gas- oder dampfförmige Desorbat nicht in die Umgebung gelangen kann.

Gelöst wird diese Aufgabe mit den Merkmalen des Anspruches 1. Vorteilhafte Ausgestaltungen des Verfahrens und eine Vorrichtung zur Durchführung des Verfahrens sind den Unteransprüchen entnehmbar.

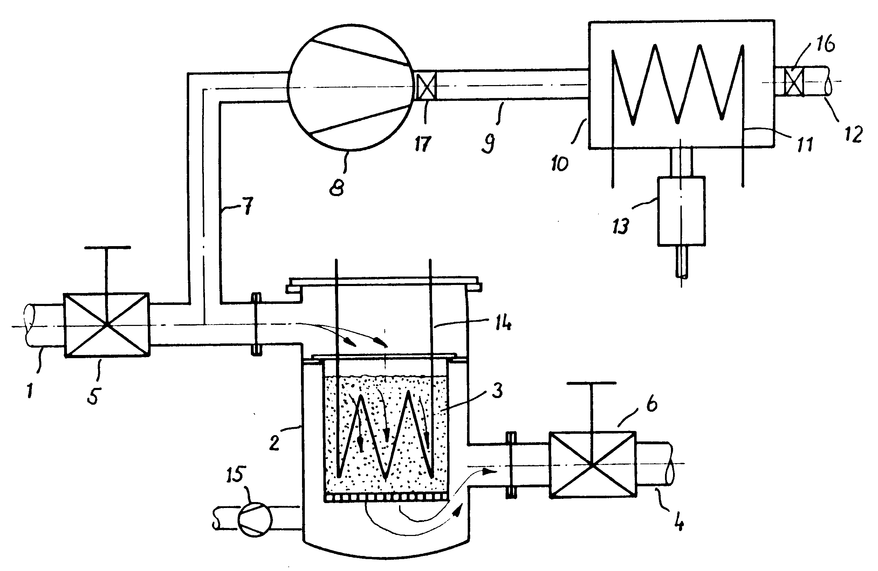

Das Verfahren und die Vorrichtung werden nachfolgend anhand der Zeichnung erläutert, welche eine Ausführungsform der Vorrichtung schematisch darstellt.

Die nachfolgende Beschreibung bezieht sich auf die Desorption von Perchloräthylen, das bei Normaldruck eine Zersetzungstemperatur von etwa 150° und einen Gefrierpunkt von -23°C aufweist.

Mit Perchloräthylen beladene Luft wird über die Leitung 1 dem Behälter 2 zugeführt, in dem sich der Adsorber 3 befindet. Bei dem Adsorbermaterial handelt es sich beispielsweise um Aktivkohle. Die mit Perchloräthylen beladene Luft durchströmt den Adsorber 3, der das Perchloräthylen adsorbiert. Die vom Perchloräthylen befreite Luft strömt sodann abluftseitig über die Rohrleitung 4 ab. In den Zu- und Abluftrohrleitungen 1,4 ist je ein Absperrschieber 5,6 angeordnet.

An die Zuluftseite ist zwischen dem Absperrschieber 5 und dem Behälter 2 an die Rohrleitung 1 eine Rohrleitung 7 angeschlossen, velche zu einer Vakuumpumpe 8 führt. Die Druckseite dieser Vakuumpumpe 8 ist über eine weitere Rohrleitung 9 mit einem Kühlbehälter 10 verbunden. Im Kühlbehälter 10 ist ein Kühler 11 angeordnet. Vom Kühlbehälter 11 kann ein Rohrstutzen 12 ins Freie verlaufen. Mit dem Kühlraum 10 ist verbunden ein Abscheider 13. Der Adsorber 3 und damit das Adsorbermaterial können mittels einer Heizvorrichtung 14 beheizt werden. Abluftseitig ist an den Behälter 2 eine Luftpumpe 15 angeschlossen.

Zum Desorbieren wird der Adsorber 3 auf eine Temperatur erhitzt, die unter der Zersetzungstemperatur des adsorbierten Stoffs liegt. Im Fall von Perchloräthylen erfolgt die Erhitzung des Adsorbers 3 auf eine Temperatur von 120°C. Nachdem das Adsorbermaterial diese Temperatur aufweist, werden die Absperrschieber 5,6 geschlossen und der Adsorberraum 2 wird von der Abluftseite her entlüftet. Dann wird die Vakuumpumpe 8 in Betrieb gesetzt, wobei mit abnehmendem Druck im Behälter 2 zunehmend mehr des adsorbierten Stoffs vom Adsorber desorbiert wird. Der Druck im Behälter 2 wird auf unter 10 mbar, bevorzugt bis 1 mbar abgesenkt. Das dampf- oder gasförmige Desorbat wird von der Vakuumpumpe 8 in den Kühlraum 10 gefördert, wo es zumindest unter die Kondensationstemperatur abgekühlt wird und somit als Kondensat anfällt. Bevorzugt erfolgt die Abkühlung des Desorbats unter seinen Gefrierpunkt. Die Abkühlung des Desorbats erfolgt auf -55°C bei einer Kühlertemperatur von -65°.

Da die Luftmenge im Bereich zwischen den Schiebern 5,6 relativ gering ist, treten vernachlässigbar geringe Mengen des Desorbats mit dieser Luft über den Rohrstutzen 12 aus.

Die Luftpumpe 15, die an die Abluftseite des Behälters 2 angeschlossen ist, dient dazu, die Luft im Bereich zwischen den Absperrschiebern 5,6 weiter zu vermindern. Diese Luftpumpe 15 wird nach dem Verschließen der Schieber 5,6 und vor Inbetriebnahme der Vakuumpumpe 8 in Betrieb gesetzt, jedoch ist der von ihr erzeugte Unterdruck im Behälter 2 nicht ausreichend, eine Desorption zu bewirken.

Im Kühlraum kondensiert das gas- bzw. dampfförmige Desorbat und erstarrt zum Teil. Soweit das Desorbat erstarrt ist, wird es nach Beendigung der Desorption des Adsorbers wieder verflüssigt und das flüssige Desorbat wird über den Abscheider 13 abgeführt. Ist Wasserdampf im gas- bzw. dampfförmigen Desorbat enthalten, wird das gebildete Wasser im Abscheider 13 abgeschieden. Das Perchloräthylen kann in flüssiger Form neu verwendet werden, z.B. zum Reinigen von Werkstücken aus Metall.

Soweit Luft im Bereich zwischen den Schiebern 5,6 vorhanden ist, die über den Rohrstutzen 12 abgeführt wird, kann im Rohrstutzen 12 ein weiterer Adsorber vorgesehen werden, der verhindert, daß geringe nicht verflüssigte Anteile des Desorbats mit dieser Luft abgeführt werden.

Die Abkühlung im Kühlraum 10 kann in zwei Stufen durchgeführt werden. In der ersten Stufe erfolgt eine Abkühlung auf eine Temperatur, die unterhalb der Kondensationstemperatur liegt, während in der zweiten Stufe auf eine Temperatur abgekühlt wird, die unter dem Gefrier- bzw. Erstarrungspunkt des Desorbats liegt.

Werden nur geringe Mengen an Luft in den Kühlraum 10 gefördert, dann ist es möglich, den Rohrstutzen 12 mittels eines Schiebers 16 zu verschließen. Der Kühlraum 10 sollte in diesem Fall ein ausreichend großes Volumen aufweisen, damit sich in ihm während des Betriebs der Vakuumpumpe 8 aufbauende Druck nicht so groß wird, daß der Unterdruck im Behälter 2 auf einen Wert ansteigt, bei dem eine Desorption nicht mehr möglich ist.

Nach Beendigung der Desorption und somit nach Stillsetzen der Vakuumpumpe 8 kann ein weiterer Schieber 17 verschlossen werden. Das Desorbat im Kühlraum 10 kann weiter abgekühlt werden, während der Adsorber 3 im Normalbetrieb arbeitet, also mit Perchloräthylen beladen wird. Hierbei kann das Desorbat im Kühlraum 10 im Kreislauf über den Kühler 11 geführt werden.

Ein wichtiges Merkmal des Verfahrens besteht darin, das Luftvolumen im Raum zwischen den Schiebern 5,6 möglichst gering zu halten. Dies wird in erster Linie durch Inbetriebnahme der Luftpumpe 15 vor Inbetriebnahme der Vakuumpumpe 8 erreicht. Bevorzugt wird auch vor Inbetriebnahme der Vakuumpumpe 8 der Schieber 16 verschlossen und während der Desorption durch den Kühler 11 so stark gekühlt, daß im Kühlraum 10 infolge der Kondensation des Desorbats sich kein nenenswerter Überdruck aufbaut. Nach dem Stillsetzen der Pumpe 8 wird sodann der Schieber 17 ebenfalls verschlossen und das im Kühl raum vorhandene Desorbat auskondensiert.

Besteht der Adsorber 3 aus Aktivkohle und besteht das Desorbat aus Perchloräthylen, muß der Unterdruck, der von der Pumpe 8 erzeugt wird, unter 10 mbar liegen. Infolge der gründlichen Desorption treten in der Leitung 4 Abluftkonzentrationen auf, die unter 20 mg/m³ liegen.

## Patentansprüche

1. Verfahren zur Rückgewinnung von in einem Adsorber (3) adsorbierten Perchloräthylen, mit folgenden zeitlich aufeinanderfolgenden Verfahrensschritten
(a) Erhitzen des Adsorbers (3) auf eine Temperatur, die unter der Zersetzungstemperatur des jeweiligen Stoffs liegt,
(b) Abschließen des Adsorberraums (2) gegenüber der Umgebung,
(c) Entlüften des Adsorberraums (2) von der Abluftseite her,
(d) Anlegen eines hohen Unterdrucks von unter 10 mbar an den abgeschlossenen Adsorberraum (2), wobei durch den Unterdruck der Stoff desorbiert wird,
(e) Abziehen des desorbierten gas- oder dampfförmigen Stoffs vom Adsorberraum (2) und Transport in einen Kühlraum (10),
(f) Abkühlen des desorbierten Stoffs im Kühlraum (10) mindestens auf eine Temperatur, bei welcher der Stoff kondensiert und bei einem Druck, der über dem Unterdruck liegt.

2. Verfahren nach Anspruch 1, **dadurch gekennzeichnet,** daß das Abkühlen auf eine Temperatur erfolgt, die unter dem Gefrierpunkt des Stoffs liegt.

3. Verfahren nach Anspruch 1 oder 2, **dadurch gekennzeichnet,** daß der desorbierte Stoff in dem Kühlraum (10) im Kreislauf über ein Kälteaggregat (11) geführt wird.

4. Verfahren nach einem der Ansprüche 1 bis 3, **dadurch gekennzeichnet,** daß die Abkühlung des desorbierten Stoffs zweistufig erfolgt, wobei in der ersten Stufe unter die Kondensationstemperatur und in der zweiten Stufe unter den Erstarrungspunkt des desorbierten Stoffs abgekühlt wird.

5. Vorrichtung zur Durchführung des Verfahrens nach einem der Ansprüche 1 bis 4, **dadurch gekennzeichnet,** daß in den Zu- und Abluftleitungen (1, 4) die zum Adsorber (3) führen, je ein Absperrschieber (5, 6) angeordnet ist, in den Adsorber (3) umschließenden Behälter (2) eine zu einer Vakuumpumpe (8) verlaufende Rohrleitung (7) mündet und von der Vakuumpumpe (8) eine weitere Rohrleitung (9) zu einem Kühlraum (10) führt, in welchem ein Kühler (11) angeordnet ist und an den Behälter (2) eine Luftpumpe (15) abluftseitig angeschlossen ist

6. Vorrichtung nach Anspruch 5, **dadurch gekennzeichnet,** daß der Kühlraum (10) ein wesentlich größeres Volumen aufweist als das zwischen den Absperrschiebern (5, 6) vorhandene Volumen und ein Schieber (17) in der weiteren Rohrleitung (9) angeordnet ist.

7. Vorrichtung nach Anspruch 5, **dadurch gekennzeichnet,** daß der Kühlraum (10) einen ins Freie mündenden Rohrstutzen (12) aufweist, der durch einen Schieber (16) abschließbar ist.

8. Vorrichtung nach Anspruch 7, **dadurch gekennzeichnet,** daß im Rohrstutzen (12) ein weiterer Adsorber angeordnet ist.

## Claims

1. A method of recovery of perchloroethylene adsorbed in an adsorber (3), by the following successive steps of the method:
(a) Heating the adsorber (3) up to a temperature which lies below the temperature of decomposition of the said substance;
(b) Closing off the adsorber chamber (2) from its surroundings;
(c) Venting the adsorber chamber (2) from the exhaust side;
(d) Applying a greatly reduced pressure of below 10 mbar to the closed adsorber chamber (2), so that the substance is desorbed through the reduced pressure;
(e) Drawing off the desorbed substance in the form of gas or vapour from the adsorber chamber (2) and conveying it into a cooling chamber (10)
(f) Cooling the desorbed substance in the cooling chamber (10) at least down to a temperature at which the substance condenses and at a pressure which lies above the reduced pressure.

2. A method as in Claim 1, characterized in that the cooling down is effected to a temperature which lies below the freezing point of the substance.

3. A method as in Claim 1 or 2, characterized in that the desorbed substance is led into the cooling chamber (10) in the circuit via a refrigerator unit (11).

4. A method as in one of the Claims 1 to 3, characterized in that the cooling dawn of the desorbed substance is effected in two stages, so that in the first stage it is cooled down below the condensation temperature and in the second stage below the solidification point of the desorbed substance.

5. A device for the performance of the method as in one of the Claims 1 to 4, characterized in that the inlet and exhaust lines (1, 4) which lead to the adsorber (3) have a gate stopvalve (5, 6) arranged in each, a pipe (7) running to a vacuum pump (8) opens into the container (2) surrounding the adsorber (3) and a further pipe (9) leads from the vacuum pump (8) to a cooling chamber (10) in which a cooler is arranged, and an airpump (15) is connected to the exhaust side of the container (2).

6. A device as in Claim 5, characterized in that the cooling chamber (10) exhibits a considerably greater volume than the volume existing between the gate stopvalves (5, 6), and a gatevalve (17) is arranged in the further pipe (9).

7. A device as in Claim 5, characterized in that the cooling chamber (10) exhibits a pipe socket (12) emerging into the open air, which may be shut off by a gatevalve (16).

8. A device as in Claim 7, characterized in that a further adsorber is arranged in the pipe socket (12).

## Revendications

1. Procédé pour récupérer du perchloréthylène adsorbé dans un adsorbeur (3), comportant les étapes successives suivantes :
(a) chauffage de l'adsorbeur (3) à une température qui est inférieure à la température de décomposition du composé considéré,
(b) fermeture du compartiment d'adsorption (2) pour l'isoler de l'environnement,
(c) dégazage du compartiment d'adsorption (2) à partir du côté effluent,
(d) application d'une forte dépression, inférieure à 10 mbar, au compartiment d'adsorption (2) ainsi obturé, le composé étant désorbé sous l'effet de la dépression,
(e) extraction, du compartiment d'adsorption (2), du côté désorbé, sous forme gazeuse ou vapeur, et transport dans un compartiment de réfrigération (10),
(f) refroidissement du composé désorbé dans le compartiment de réfrigération (10), au moins à une température à laquelle le composé se condense et sous une pression supérieure à la dépression.

2. Procédé selon la revendication 1, caractérisé en ce que le refroidissement s'effectue à une température inférieure au point de congélation du composé.

3. Procédé selon la revendication 1 ou 2, caractérisé en ce que le composé désorbé dans le compartiment de réfrigération (10) est envoyé en circulation en circuit fermé par l'intermédiaire d'un groupe frigorifique (11).

4. Procédé selon l'une des revendications 1 à 3, caractérisé en ce que le refroidissement du composé désorbé s'effectue en deux étapes, le refroidissement de la première étape s'effectuant en dessous de la température de condensation, et le refroidissement de la deuxième étape s'effectuant en dessous du point de solidification du composé désorbé.

5. Dispositif destiné à mettre en oeuvre le procédé selon l'une quelconque des revendications 1 à 4, caractérisé en ce qu'on dispose, dans chacune des conduites d'amenée et d'évacuation de l'air (1, 4) qui vont à l'adsorbeur, une vanne d'arrêt (5, 6), en ce qu'une tuyauterie (7), courant vers une pompe à vide (8), débouche dans l'enceinte (2) qui entoure l'adsorbeur (3), et qu'une autre tuyauterie (10), partant de la pompe à vide (8), va vers un compartiment de réfrigération (10) dans lequel est disposé un dispositif réfrigérateur (11), une pompe à air (15) étant raccordée, côté air d'évacuation, à l'enceinte (2).

6. Dispositif selon la revendication 5, caractérisé en ce que le compartiment de réfrigération (10) a un volume nettement plus grand que le volume présent entre les vannes d'arrêt (5, 6), une vanne (17) étant disposée dans la suite de la tuyauterie (9).

7. Dispositif selon la revendication 7, caractérisé en ce que le compartiment de réfrigération (10) comporte une tubulure (12) qui débouche à l'air libre et qui peut être obturée à l'aide d'une vanne (16).

8. Dispositif selon la revendication 7, caractérisé en ce qu'un adsorbeur supplémentaire est disposé dans la tubulure (12).
